# EUROPEAN PATENT SPECIFICATION

(11) **EP 3 517 711 B1**
(45) Date of publication and mention of the grant of the patent: **08.07.2020**
(21) Application number: 18709055.0
(22) Date of filing: 05.01.2018
(51) Int. Cl.: E05B 1/00, E05B 5/00, E05C 9/02

(54) **WINDOWS AND DOORS OPENING AND CLOSING MECHANISM**
MECHANISMUS ZUM ÖFFNEN UND SCHLIESSEN VON FENSTERN UND TÜREN
MÉCANISME D'OUVERTURE ET DE FERMETURE DE FENÊTRES ET PORTES

(30) Priority: 13.01.2017 ES 201730029 U
(43) Date of publication of application: 31.07.2019
(73) Proprietor: Sistemas Técnicos Del Accesorio Y Componentes, S.L. (STAC), 15900 Padrón (A Coruña) (ES)
(72) Inventor: CASTRO SOMOZA, José, 15900 Padrón (A Coruña) (ES); PIÑEIRO CASTELO, Marcos, 15900 Padrón (A Coruña) (ES); FERNÁNDEZ COBIÁN, Javier, 15900 Padrón (A Coruña) (ES); FERNÁNDEZ PADRÓN, Juan Carlos, 15900 Padrón (A Coruña) (ES)
(74) Representative: Pons
(86) International application number: PCT/ES2018/070006
(87) International publication number: WO 2018/130733

(56) References cited:
- EP-A1- 3 018 269
- CN-A- 102 345 423
- US-A1- 2006 244 269
- US-A1- 2010 132 262
- US-B1- 6 425 611

## Description

### OBJECT OF THE INVENTION

The present invention relates to a windows and doors opening and closing mechanism. It can be included in the technical field of manufacturing windows and doors and the fittings thereof.

More specifically, the object of the invention is an opening and closing mechanism wherein the elements that are normally visible, such as the handle, are hidden. Said elements are housed in a hollow of the profile of the window or door by which the aesthetics of the mechanism are improved and the risk of user contact with the base of the handle is prevented when performing the movements for opening or closing the window/door.

### BACKGROUND OF THE INVENTION

Windows and doors comprise a fixed profile called a "frame profile" and a mobile profile called a "sash profile". All the active elements that enable the window/door to be opened or closed are located inside the mobile profile.

Currently, the windows and doors that are known in the market have closing systems with handles and espagnolettes. They generally include a manual actuating handle in order to transmit the rotating movement to the inner mechanisms of the system located in the mobile profile, whether they be transmission boxes in the case of handles or transmitting sleeves in the case of espagnolettes, etc.

The rotating movement transmitted by the handle is transformed into translation movement by mechanisms that can be both inside the mobile profile in the form of a transmission box, and at the base of the espagnolette handle, both with rack and pinion systems. Patents CH485099, DE1815345, DE3445170A1 and EP0446566 disclose said systems.

However, market developments are moving towards mechanisms that are partially hidden in the actuating handle itself or mechanisms hidden inside the mobile profile. Some of the documents that describe this type of partially hidden systems are ES1148208U, 1146109U, DE4231123A1, DE4238459A1, DE9418857U1, DE19541214C1, WO2015198171A1 and EP1903165A2.

Documents EP3018269 and US6425611 disclose also pivoting handles that are partially hidden inside the frame of doors or windows.

Document CN102345423 shows a multi-point lock arrangement comprising a casing which can be inserted inside a lateral surface of a window or door. In this disclosure the lock is operated by means of a sliding actuating element.

### DESCRIPTION OF THE INVENTION

The present invention describes a mechanism for opening and closing windows and doors configured to be housed in a lateral of the mobile profile of the window/door. Thus, the mechanism is completely invisible to the user from a front viewpoint of the window/door. In this way, the visual impact is almost nonexistent and this favors the minimalist aesthetics of the window/door.

The mobile profiles of windows/doors comprise inner fittings and transmitting bars in order to carry out the corresponding sliding for opening and closing the window/door. The proposed mechanism comprises at least one handle that moves with a circular movement and a rack that moves with a linear movement as a consequence of the circular movement of the handle. The rack is located on the sliding track of the fitting and of the transmitting bars of the mobile profile of the window/door.

In order to transmit the circular movement of the handle to a linear movement of the rack, the handle is a type of cam and comprises at one end, on which it rotates, a circular toothed segment that meshes with the teeth of the rack. In another exemplary embodiment the movement of the handle can be linear. In this case, the transmission of the linear movement that the user exerts on the handle is made directly to the rack.

The rack can be directly displaceable or it can be mounted on guides that move. In both cases the displacement is carried out on the sliding track of the fitting and of the transmitting bars of the mobile profile. Thus, the linear movement is transmitted to the other elements of the window or door that enable opening or closing thereof.

The elements of the mechanism are housed in the mobile profile such that they are completely protected and hidden from the user's view. The mechanism comprises a casing that is housed in the hollow of the profile and in which the handle is also found. Thus, the handle, which is the element used to act on the mechanism and to move the window/door during opening or closing, is also hidden. More specifically, the handle stays flush with the casing in the mobile profile.

In the mechanism of the present invention, the handle moves between three positions. A first position corresponds to one of the types of opening of the window/door and is one of the end positions of the handle, in which it is flush with the casing and therefore with the mobile profile.

A second position corresponds to an intermediate position, where the handle can be used as a knob to make it easier for the user to move the window/door when opening or closing it. In this second position the handle is arranged with a certain angle with respect to the lateral of the mobile profile.

A third position corresponds to the closing of the window/door and is the other end position of the handle. In this case it again stays flush with the casing and the mobile profile. Thus, it is hidden from the user's view again.

The mechanism comprises a retention device, arranged in the casing, in order to ensure the three positions of the handle. In a first exemplary embodiment the retention device comprises a strip with a projection intended to be housed in recesses of the handle, which correspond to each of the positions thereof. In a second exemplary embodiment the retention device comprises a flat strip that limits the movement of the handle when coming into contact with flat faces thereof.

Furthermore, in the second exemplary embodiment, the opening and closing mechanism can comprise an intermediate element for transmission of the movement between the handle and the rack. Preferably, said intermediate element is a pinion that, when actuated by the rotating movement of the handle, moves with a rotating movement in the opposite direction. In turn, the rotating movement of the pinion is what causes the displacement of the rack.

### DESCRIPTION OF THE DRAWINGS

As a complement to the description provided herein and for the purpose of helping to make the characteristics of the invention more readily understandable, in accordance with a preferred practical embodiment thereof, said description is accompanied by a set of drawings constituting an integral part of the same, which by way of illustration and not limitation represent the following:
Figure 1 shows a view of a section of a mobile profile, and the front direction of the window and the front direction of the mobile profile have been indicated to show the position in which the opening and closing mechanism is installed.
Figure 2 shows a view of the lateral of the mobile profile in which the opening and closing mechanism is seen.
Figures 3a-3b show views of the opening and closing mechanism in which the movements of the handle and the rack have been indicated with arrows.
Figures 4a-4b show views of an exemplary embodiment of the retention device.
Figures 5a-5b show views of another exemplary embodiment of the retention device.
Figure 6 shows an exploded view of the opening and closing mechanism in a first embodiment.
Figure 7 shows a perspective view of the opening and closing mechanism of the first embodiment.
Figure 8 shows a view in which the retention device is shown and how the handle and the rack are linked.
Figure 9 shows a cross-section view of the opening and closing mechanism of the first embodiment.
Figure 10 shows an exploded view of the opening and closing mechanism in a second embodiment.
Figure 11 shows a perspective view of the opening and closing mechanism of the second embodiment.
Figure 12 shows a view in which the retention device can be seen and how the handle, the pinion and the rack are linked.
Figure 13 shows a cross-section view of the opening and closing mechanism of the second embodiment.

### PREFERRED EMBODIMENT OF THE INVENTION

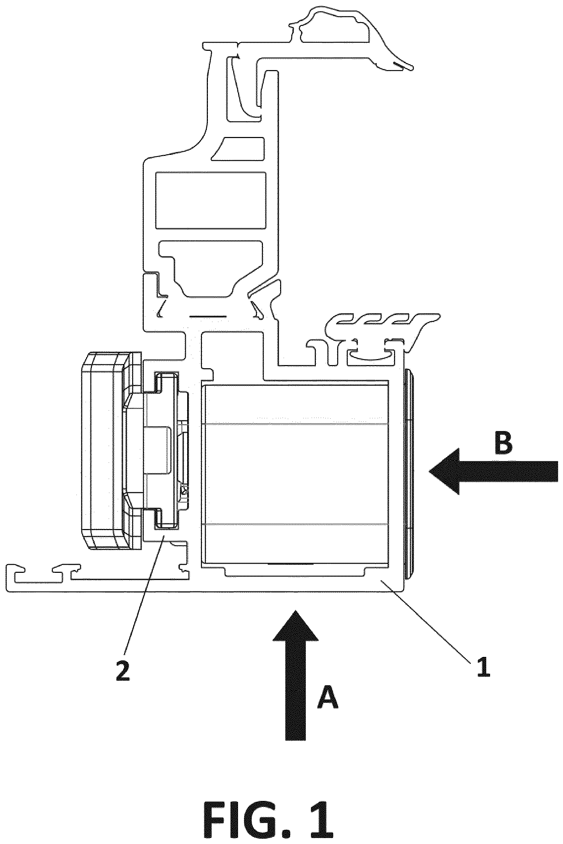

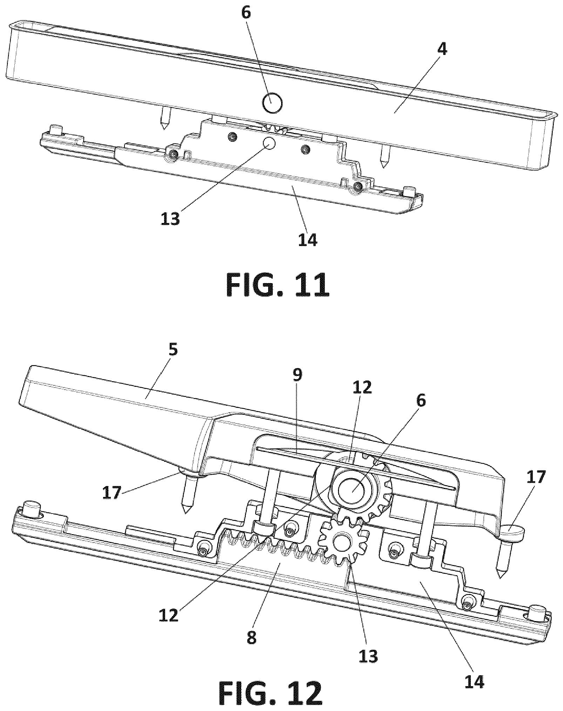

What follows is a description, with the help of Figures 1 to 13, of examples of embodiment of the invention.

The described mechanism for opening and closing windows and doors is designed to be housed in a hollow (3) in a lateral of the mobile profile (1) of the window or door in which it is installed. The mechanism is designed such that all the elements thereof are hidden from view when a user observes the window/door from a frontal position (A).

Figure 1 shows a sectional view of the mobile profile (1) of a window in which the opening and closing mechanism of the invention has been installed. As seen in the figure, the mechanism is installed in a lateral of the mobile profile (1) such that a user can only see said mechanism when looking at the window (or door, where applicable) from a lateral position (B).

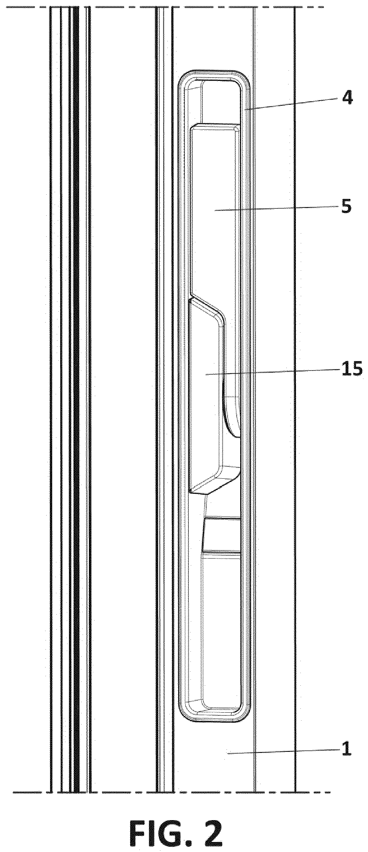

Figure 2 shows a view of the mobile profile (1) with the opening and closing mechanism. Said view is the image that the user sees when looking at the window/door from the lateral position (B) indicated in Figure 1.

The opening and closing mechanism is configured to be installed in windows/doors of the type that comprise a fitting and transmitting bars, and a sliding track (2) of the fitting as seen in Figure 1.

The mechanism comprises at least one casing (4) intended to be housed in the hollow (3) of the mobile profile (1) and joined thereto. It also comprises at least one handle (5) that can be rotated around a shaft (6) through which it is linked to the casing (4). The handle (5) comprises, on one of the ends thereof, at least one circular toothed segment (7) and that can be moved between three positions.

The casing (4) is joined to the mobile profile (1) by means of a fastening system comprising metal inserts, introduced through mechanized holes in the mobile profile (1), and screws. This fastening system causes a pressure between the casing (4) and the mobile profile (1), which provides a rigid and resistant connection to ensure the correct operation of the handle (5).

A first position of the handle (5) is an open position of the window or door, in which the handle (5) stays flush with the casing (4) and with the surface of the mobile profile (1).

A second position of the handle (5) is an intermediate position, in which the handle (5) is arranged at a certain angle from the surface of the mobile profile (1) and said handle (5) can act as a knob in order to enable the displacement of the mobile profile (1) of the window or door.

A third position of the handle (5) is a closed position of the window or door, in which the handle (5) stays flush with the casing (4) in a position symmetrical to the first position.

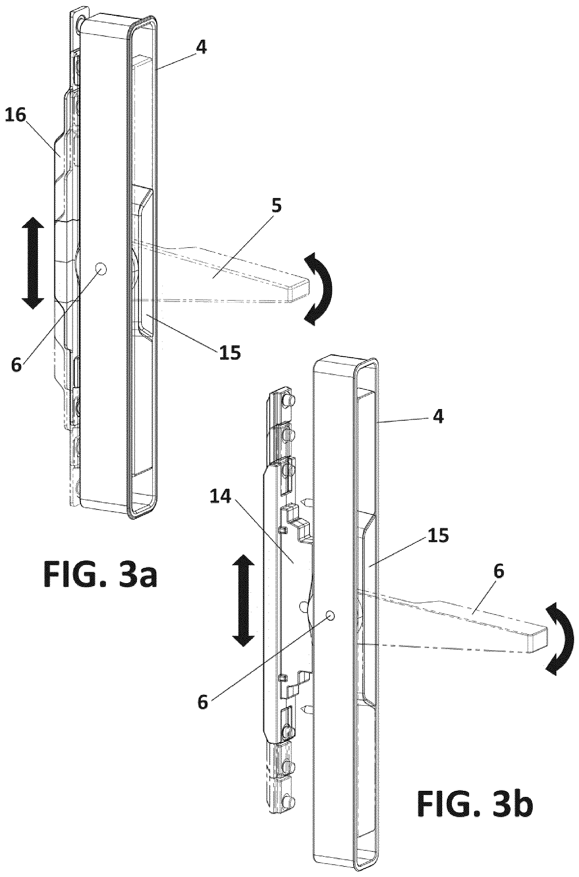

Figures 3a and 3b show the opening and closing mechanism in two different embodiments. In both cases the movement of the handle (5) that causes the displacement thereof between the three previously described positions is shown by arrows.

The mechanism also comprises a rack (8), which can be displaced linearly, as a consequence of the rotating movement of the handle (5) through the sliding track for opening or closing the window or door. Figures 3a and 3b also show the linear movement of the rack (8) in each of the two embodiments.

Likewise, the mechanism preferably comprises a retention device, for ensuring the positions of the handle (5). This retention device comprises at least one strip (9) joined to the casing (4) and which is in contact with the end of the handle (5) that is joined to the shaft (6).

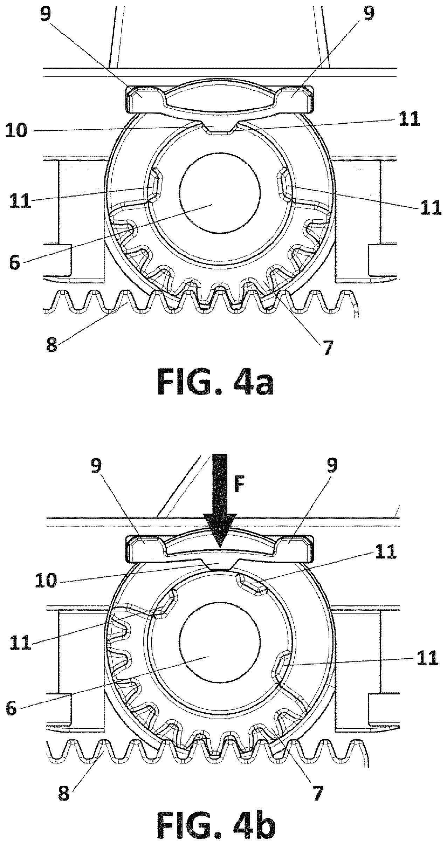

In an exemplary embodiment, which is seen in Figures 4a and 4b, the strip (9) comprises a projection (10) configured to be housed in recesses (11) provided in the handle (5) on the end that is joined to the shaft (6) and which fasten the positions thereof.

Figure 4a shows the projection (10) of the strip (9) housed in a notch (11) of the handle (5). Figure 4b shows how, when turning the handle (5), the projection (10) that was fastened in the described position (housed in one of the notches (11)) is released. An arrow is represented with the direction in which the force (F) is exerted, which is the recovery force of the strip (9) towards the initial position thereof and which is aimed towards the notches (11).

This force is overcome only when the user is turning the handle (5). When the user has turned the handle (5) to the desired position, it stops moving and the strip (9), with the recovery force (F), returns to the resting position thereof and, if the handle (5) is well-positioned in the path thereof, the projection (10) will be facing the notch (11) and by said recovery force (F) it will be housed in the notch (11).

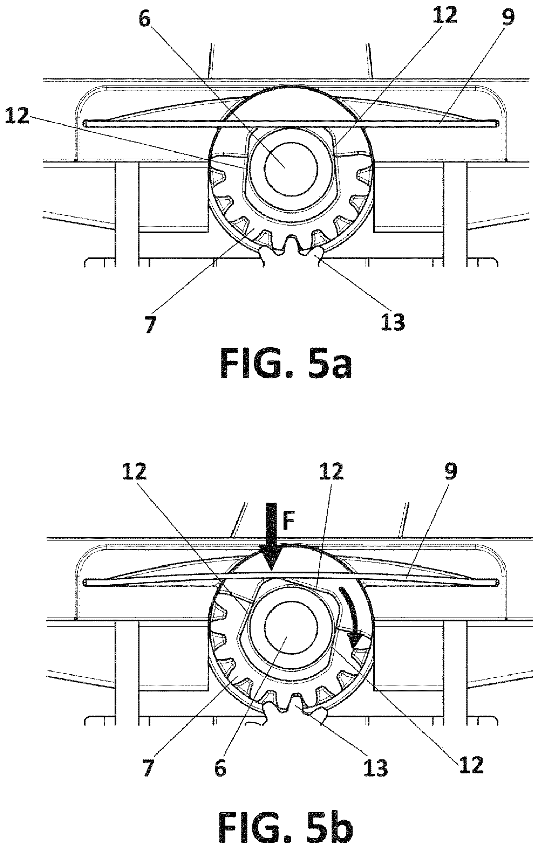

In another exemplary embodiment shown in Figures 5a and 5b, the strip (9) is a flat strip and the handle (5) comprises, on the end thereof that is joined to the shaft (6), flat faces (12) such that the strip (9) comes into contact with said flat faces (12), fastening the positions of the handle (5).

Figure 5a shows the strip (9) in an upright position, abutting on a flat face (12) of the handle (5), which marks one of the positions of the handle (5). Figure 5b shows the rotating movement of the handle (5) as well as the direction of the recovery force (F) of the strip (9).

As seen in said Figure 5b, in this exemplary embodiment, the handle (5), on the end that is joined to the shaft (6), comprises three flat faces (12), each of them to delimit one of the positions of the handle (5). When the handle (5) is in one of the described positions, the strip (9) abuts on the corresponding flat face (12).

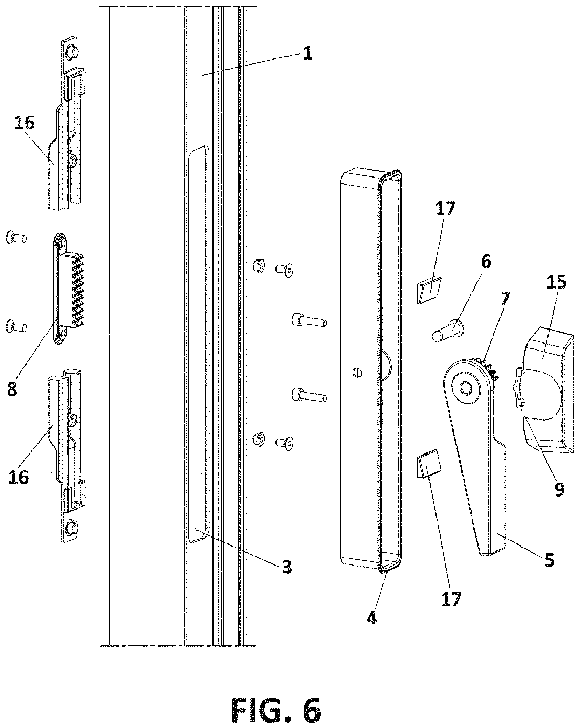

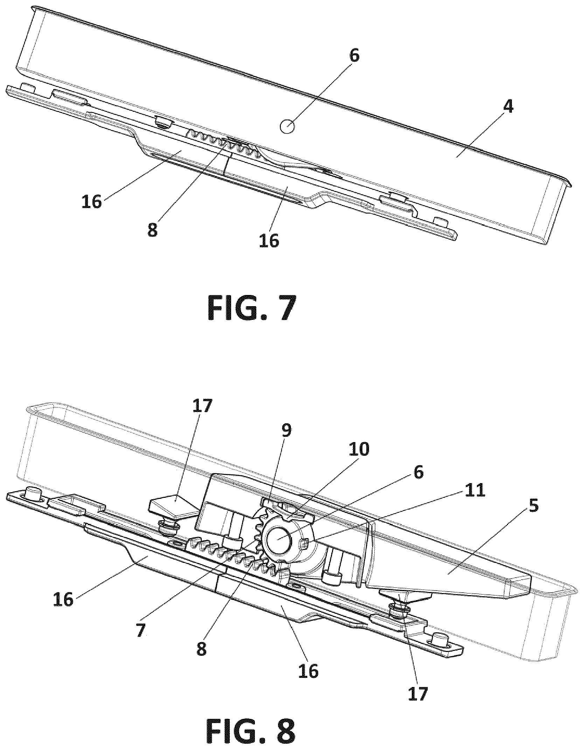

Figure 6 shows an exploded view of the mechanism for opening and closing windows and doors, in an embodiment in which the handle (5) is directly connected to the rack (8). Figures 7 to 9 also represent this same embodiment.

In this embodiment, the rack (8) is anchored by means of screws to two grooved symmetrical slides (16) in the sliding track of the fitting and of the transmitting bars of the mobile profile, that altogether enable the linear movement of the rack to be transmitted to the other devices of the window or door that enable the opening or closing thereof.

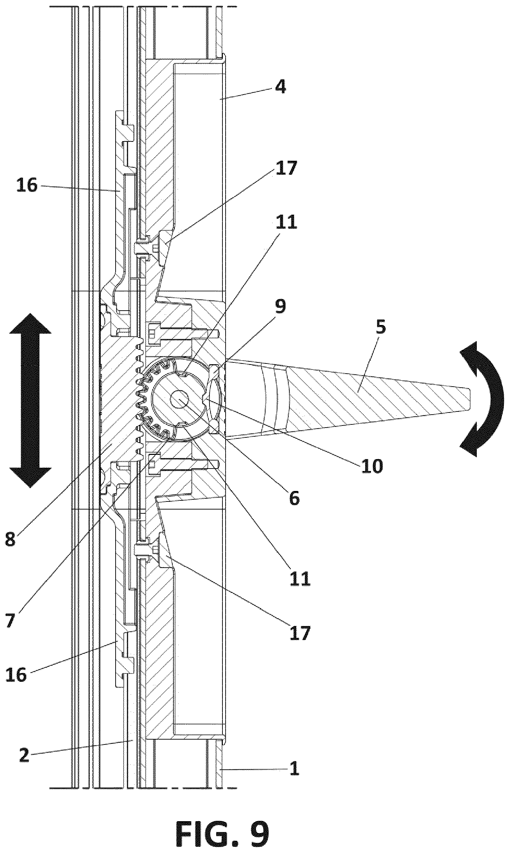

Likewise, as seen in the figures, the mechanism can comprise a cover (15) arranged in the casing (4) configured to cover the circular toothed segment (7) of the handle (5). In an exemplary embodiment, as seen for example in Figure 9, the strip (9) is arranged in said cover (15).

Figure 7 shows a perspective view of the opening and closing mechanism in which the casing (4), the rack (8) and the symmetrical slides (16) can be seen. Figure 8 shows the elements inside the casing (4) and shows how the handle (5) and the rack (8) are linked by the circular toothed segment (7). Figure 9 also shows how the circular movement of the handle (5) is transformed into a linear movement of the rack (8).

Additionally the mechanism can comprise elastic stops (17) housed in the casing (4) in order to prevent the impact of the handle (5) against the casing (4) in the end positions of the path of the handle (5). Thus, unpleasant sounds caused by said impacts are prevented. Figure 6 shows the elastic stops (17) in the exploded view of the mechanism and Figures 8 and 9 show the position thereof in the casing (4). These elastic stops (17) can also act as trims by covering the fastenings to the mobile profile (1).

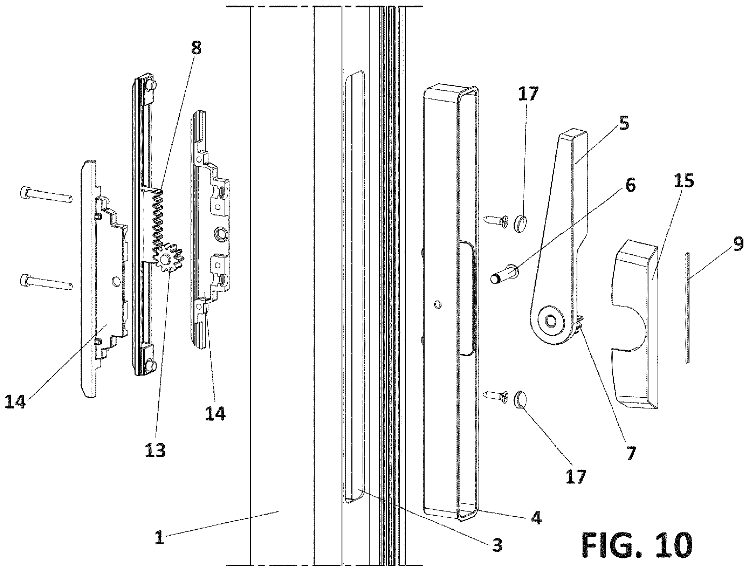

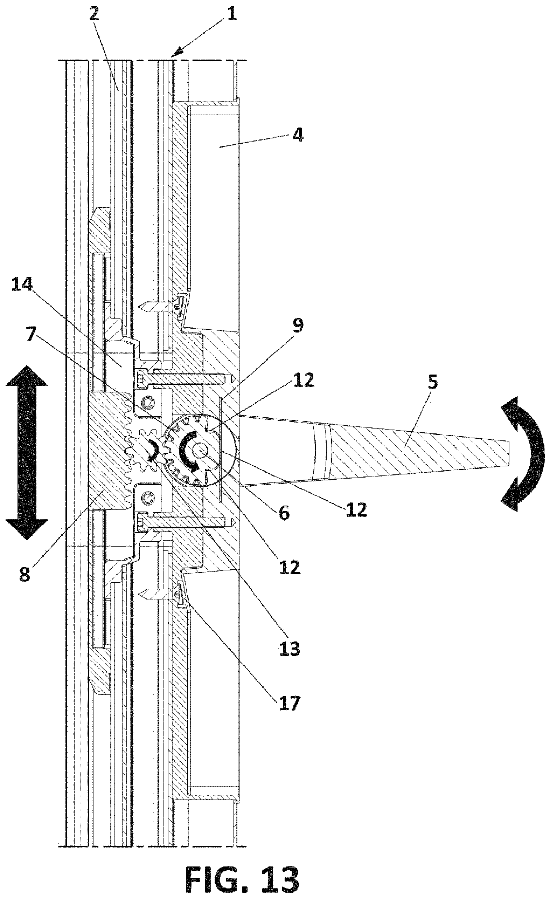

Another embodiment of the invention is shown in Figures 10 to 13. In this case the mechanism further comprises a pinion (13) arranged between the circular toothed segment (7) and the rack (8). Thus, the circular movement of the handle (5) causes the rotation, in the opposite direction, of the pinion (13), which in turn causes the linear displacement of the rack (8). In Figure 13 the movements of the different elements of the mechanism have been represented by arrows.

The mechanism can also comprise an inner casing (14) to which the pinion (13) is joined and inside which the rack (8) is housed. This inner casing (14) enables the gear of the pinion (13) with the rack (8) to be protected.

In this embodiment, the mechanism can also comprise elastic stops (17) like those described previously. Said elastic stops (17) are seen in the position thereof in the casing (4) in Figure 13.

## Claims

1. A windows and doors opening and closing mechanism configured to be installed in a lateral of the mobile profile (1) of a window or door of the type comprising:
- a fitting and transmitting bars, and
- a sliding track (2) of the fitting;
- a hollow (3) in one of the laterals thereof;
the opening and closing mechanism comprising:
- a casing (4) intended to be housed in the hollow (3) of the mobile profile (1) and joined thereto;
- a handle (5) that can be rotated around a shaft (6) through which it is linked to the casing (4) and comprising, on one of the ends thereof, at least one circular toothed segment (7) and that can be moved between:
- a first position which is an open position of the window or door;
- a second position which is an intermediate position, in which the handle (5) is arranged at a certain angle from the surface of the lateral of the mobile profile (1);
- a rack (8), which can be displaced linearly, as a consequence of the rotating movement of the handle (5) through the sliding track for opening or closing the window or door,
**characterized in that**:
- in the first position the handle (5) stays flush with the casing (4) and with the surface of the lateral of the mobile profile (1), and
- the handle can be moved into a third position which is a closed position of the window or door, in which the handle (5) stays flush with the casing (4) in a position symmetrical to the first position.

2. The windows and doors opening and closing mechanism according to claim 1, **characterized in that** it comprises a retention device for ensuring the positions of the handle (5), comprising at least one strip (9) joined to the casing (4) and which is in contact with the end of the handle (5) that is joined to the shaft (6).

3. The windows and doors opening and closing mechanism according to claim 2, **characterized in that** the strip (9) comprises a projection (10) configured to be housed in recesses (11) provided in the handle (5) on the end that is joined to the shaft (6) and which fasten the positions thereof.

4. The windows and doors opening and closing mechanism according to claim 2, **characterized in that** the strip (9) is a flat strip and the handle (5) comprises, on the end thereof that is joined to the shaft (6), flat faces (12) such that the strip (9) comes into contact with said flat faces (12), fastening the positions of the handle (5).

5. The windows and doors opening and closing mechanism according to claim 1, **characterized in that** it further comprises a pinion (13) arranged between the circular toothed segment (7) and the rack (8) such that the circular movement of the handle (5) causes the rotation, in the opposite direction, of the pinion (13), which in turn causes the linear displacement of the rack (8).

6. The windows and doors opening and closing mechanism according to claim 5, **characterized in that** it further comprises an inner casing (14) to which the pinion (13) is joined and inside which the rack (8) is housed.

7. The windows and doors opening and closing mechanism according to claim 1, **characterized in that** it further comprises a cover (15) arranged in the casing (4) configured to cover the circular toothed segment (7) of the handle (5).

8. The windows and doors opening and closing mechanism according to claim 7, **characterized in that** the strip (9) is arranged in the cover (15).

9. The windows and doors opening and closing mechanism according to claim 1, **characterized in that** it further comprises grooved symmetrical slides (16) configured to be moved along the sliding track (2) of the fitting.

## Patentansprüche

1. Öffnungs- und Schließmechanismus für Fenster und Türen, der konfiguriert ist, um in einem Seitenteil des mobilen Profils (1) eines Fensters oder einer Tür des Typs installiert zu werden, der Folgendes umfasst:
- einen Beschlag und Übertragungsstangen und
- eine Gleitschiene (2) des Beschlags;
- eine Vertiefung (3) in einem der Seitenteile davon;
wobei der Öffnungs- und Schließmechanismus Folgendes umfasst:
- ein Gehäuse (4), das in der Vertiefung (3) des mobilen Profils (1) untergebracht und damit verbunden werden soll;
- einen Griff (5), der um eine Welle (6) gedreht werden kann, durch die er mit dem Gehäuse (4) verbunden ist und an einem seiner Enden mindestens ein kreisförmiges Zahnsegment (7) umfasst und der bewegt werden kann zwischen:
- einer ersten Position, die eine offene Position des Fensters oder der Tür ist;
- einer zweiten Position, die eine Zwischenposition ist, in der der Griff (5) in einem bestimmten Winkel von der Oberfläche des Seitenteils des mobilen Profils (1) angeordnet ist;
- eine Zahnstange (8), die infolge der Drehbewegung des Griffs (5) durch die Gleitschiene zum Öffnen oder Schließen des Fensters oder der Tür linear verschoben werden kann,
**dadurch gekennzeichnet, dass**:
- in der ersten Position der Griff (5) bündig mit dem Gehäuse (4) und mit der Oberfläche der Seite des mobilen Profils (1) bleibt, und
- der Griff in eine dritte Position bewegt werden kann, die eine geschlossene Position des Fensters oder der Tür ist, in der der Griff (5) in einer Position symmetrisch zur ersten Position bündig mit dem Gehäuse (4) bleibt.

2. Öffnungs- und Schließmechanismus für Fenster und Türen nach Anspruch 1, **dadurch gekennzeichnet, dass** er eine Rückhaltevorrichtung zum Sicherstellen der Positionen des Griffs (5) umfasst, die mindestens einen Streifen (9) umfasst, der mit dem Gehäuse (4) verbunden ist und der mit dem Ende des Griffs (5), der mit der Welle (6) verbunden ist, in Kontakt steht.

3. Öffnungs- und Schließmechanismus für Fenster und Türen nach Anspruch 2, **dadurch gekennzeichnet, dass** der Streifen (9) einen Vorsprung (10) umfasst, der konfiguriert ist, in Aussparungen (11) untergebracht zu sein, die in dem Griff (5) an dem Ende bereitgestellt sind, das mit der Welle (6) verbunden ist und das die Positionen davon fixiert.

4. Öffnungs- und Schließmechanismus für Fenster und Türen nach Anspruch 2, **dadurch gekennzeichnet, dass** der Streifen (9) ein flacher Streifen ist und der Griff (5) an seinem Ende, das mit der Welle (6) verbunden ist, flache Flächen (12) umfasst, sodass der Streifen (9) mit den flachen Flächen (12) in Kontakt kommt und die Positionen des Griffs (5) fixiert werden.

5. Öffnungs- und Schließmechanismus für Fenster und Türen nach Anspruch 1, **dadurch gekennzeichnet, dass** er ferner ein Ritzel (13) umfasst, das zwischen dem kreisförmigen Zahnsegment (7) und der Zahnstange (8) derart angeordnet ist, dass die kreisförmige Bewegung des Griffs (5) die Drehung des Ritzels (13) in die entgegengesetzte Richtung bewirkt, was wiederum die lineare Verschiebung der Zahnstange (8) bewirkt.

6. Öffnungs- und Schließmechanismus für Fenster und Türen nach Anspruch 5, **dadurch gekennzeichnet, dass** er ferner ein Innengehäuse (14) umfasst, mit dem das Ritzel (13) verbunden ist und in dem die Zahnstange (8) untergebracht ist.

7. Öffnungs- und Schließmechanismus für Fenster und Türen nach Anspruch 1, **dadurch gekennzeichnet, dass** er ferner eine Abdeckung (15) umfasst, die in dem Gehäuse (4) angeordnet ist, das konfiguriert ist, um das kreisförmige Zahnsegment (7) des Griffs (5) abzudecken.

8. Öffnungs- und Schließmechanismus für Fenster und Türen nach Anspruch 7, **dadurch gekennzeichnet, dass** der Streifen (9) in der Abdeckung (15) angeordnet ist.

9. Öffnungs- und Schließmechanismus für Fenster und Türen nach Anspruch 1, **dadurch gekennzeichnet, dass** er ferner gerillte symmetrische Schieber (16) umfasst, die konfiguriert sind, um entlang der Gleitschiene (2) des Beschlags bewegt zu werden.

## Revendications

1. Mécanisme d'ouverture et de fermeture de fenêtres et de portes configuré pour être installé sur un côté du profilé mobile (1) d'une fenêtre ou porte du type comprenant :
- une ferrure et des barres de transmission, et
- une piste de coulissement (2) de la ferrure ;
- un creux (3) dans l'un de ses côtés ;
le mécanisme d'ouverture et de fermeture comprenant :
- un boîtier (4) destiné à être logé dans le creux (3) du profilé mobile (1) et relié à celui-ci ;
- une poignée (5) qui peut être tournée autour d'un arbre (6) à travers lequel elle est liée au boîtier (4) et comprenant, à l'une de ses extrémités, au moins un segment denté circulaire (7) et qui peut être déplacée entre :
- une première position qui est une position ouverte de la fenêtre ou porte ;
- une deuxième position qui est une position intermédiaire, dans laquelle la poignée (5) est disposée à un certain angle depuis la surface du côté du profilé mobile (1) ;
- une crémaillère (8), qui peut être déplacée linéairement, suite au mouvement de rotation de la poignée (5) à travers la piste de coulissement pour ouvrir ou fermer la fenêtre ou porte,
**caractérisé en ce que** :
- dans la première position la poignée (5) reste au ras du boîtier (4) et de la surface du côté du profilé mobile (1), et
- la poignée peut être déplacée dans une troisième position qui est une position fermée de la fenêtre ou porte, dans laquelle la poignée (5) reste au ras du boîtier (4) dans une position symétrique à la première position.

2. Mécanisme d'ouverture et de fermeture de fenêtres et de portes selon la revendication 1, **caractérisé en ce qu'**il comprend un dispositif de retenue pour assurer les positions de la poignée (5), comprenant au moins une bande (9) reliée au boîtier (4) et qui est en contact avec l'extrémité de la poignée (5) qui est reliée à l'arbre (6).

3. Mécanisme d'ouverture et de fermeture de fenêtres et de portes selon la revendication 2, **caractérisé en ce que** la bande (9) comprend une saillie (10) configurée pour être logée dans des évidements (11) prévus dans la poignée (5) à l'extrémité qui est reliée à l'arbre (6) et qui fixent ses positions.

4. Mécanisme d'ouverture et de fermeture de fenêtres et de portes selon la revendication 2, **caractérisé en ce que** la bande (9) est une bande plate et la poignée (5) comprend, à son extrémité qui est reliée à l'arbre (6), des faces planes (12) de sorte que la bande (9) entre en contact avec lesdites faces planes (12), fixant les positions de la poignée (5).

5. Mécanisme d'ouverture et de fermeture de fenêtres et de portes selon la revendication 1, **caractérisé en ce qu'**il comprend en outre un pignon (13) disposé entre le segment denté circulaire (7) et la crémaillère (8) de sorte que le mouvement circulaire de la poignée (5) provoque la rotation, dans le sens opposé, du pignon (13), qui provoque à son tour le déplacement linéaire de la crémaillère (8).

6. Mécanisme d'ouverture et de fermeture de fenêtres et de portes selon la revendication 5, **caractérisé en ce qu'**il comprend en outre un boîtier interne (14) auquel le pignon (13) est relié et à l'intérieur duquel la crémaillère (8) est logée.

7. Mécanisme d'ouverture et de fermeture de fenêtres et de portes selon la revendication 1, **caractérisé en ce qu'**il comprend en outre un couvercle (15) disposé dans le boîtier (4) configuré pour couvrir le segment denté circulaire (7) de la poignée (5).

8. Mécanisme d'ouverture et de fermeture de fenêtres et de portes selon la revendication 7, **caractérisé en ce que** la bande (9) est disposée dans le couvercle (15).

9. Mécanisme d'ouverture et de fermeture de fenêtres et de portes selon la revendication 1, **caractérisé en ce qu'**il comprend en outre des glissières (16) symétriques rainurées configurées pour être déplacées le long de la piste de coulissement (2) de la ferrure.
